# EUROPEAN PATENT SPECIFICATION

(11) **EP 0 652 951 B1**
(45) Date of publication and mention of the grant of the patent: **01.09.1999**
(21) Application number: 91915864.2
(22) Date of filing: 14.06.1991
(51) Int. Cl.: C12N 15/12, C07K 2/00

(54) **GDF-1and UOG-1 proteins**
GDF-1 und UOG1 Proteine
Proteines GDF-1 et UOG-1

(30) Priority: 15.06.1990 US 538372; 16.11.1990 US 614452
(43) Date of publication of application: 17.05.1995
(73) Proprietor: CARNEGIE INSTITUTION OF WASHINGTON, Washington, D.C. 20005 (US)
(72) Inventor: LEE, Se-Jin, Baltimore, MD 21209 (US)
(74) Representative: Smart, Peter John
(86) International application number: US9104096
(87) International publication number: WO9200382

(56) References cited:
- US-A- 4 886 747
- MOLECULAR ENDOCRINOLOGY vol. 4, no. 7, July 1990, pages 1034 - 1040 SE-JIN LEE 'Identification of a novel member (GDF-1) of the transforming growth factor-beta superfamily'
- The EMBO Journal, Volume 9, No. 7, issued July 1990, E. OZKAYNAK et al., "OP-1 cDNA Encodes an Osteogenic Protein in the TGF-Beta Family", pages 2085-2093, see entire document.
- Proceedings of the National Academy of Sciences, Vol. 88, issued May 1991, LEE, "Expression of Growth/Differentiation Factor 1 in the Nervous System: Conservation of a Bicistronic Structure", pages 4250-4254, see entire document.
- Proceedings of the National Academy of Sciences, Vol. 86, issued June 1989, LYONS et al., "Vgr-1, a Mammalian Gene Related to Xenopus VG-1, is a Member of the Transforming Growth Factor Beta Gene Superfamily", pages 4554-4558, see entire document.

## Description

The present invention relates, in general, to DNA segments encoding proteins of the transforming growth factor beta superfamily. In particular, the present invention relates to a mammalian GDF-1 protein and to a DNA segment encoding GDF-1, and unique fragments thereof. The invention further relates to a mammalian UOG-1 protein and to a DNA segment encoding same and fragments thereof.

A growing number of polypeptide factors playing critical roles in regulating differentiation processes during embryogenesis have been found to be structurally homologous to transforming growth factor β (TGF-β). Among these are Mullerian inhibiting substance (MIS) [Cate et al, Cell 45:685-698 (1986)], which causes regression of the Mullerian duct during male sex differentiation; the bone morphogenetic proteins (BMP's) [Wozney et al, Science 242:1528-1534 (1988)], which can induce de novo cartilage and bone formation; the inhibins and activins [Mason et al, Nature 318:659-663 (1985); Forage et al, Proc. Natl. Acad. Sci., USA 83:3091-3095 (1986); Eto et al, Biochem Biophys Res Comm 142:1095-1103 (1987); and Murata et al, Proc. Natl. Acad. Sci. USA 85:2434-2438 (1988)], which regulate secretion of follicle-stimulating hormone by pituitary cells and which, in the case of the activins, can affect erythroid differentiation; the Drosophila decapentaplegic (DPP) gene product [Padgett et al, Nature 325:81-84 (1987)], which influences dorsal-ventral specification as well as morphogenesis of the imaginal disks; the Xenopus Vg-1 gene product [Weeks et al, Cell 51:861-867 (1987)], which localizes to the vegetal pole of eggs; and Vgr-1 [Lyons et al, Proc. Natl. Acad. Sci., USA 86:4554-4558 (1989)], a gene identified on the basis of its homology to Vg-1 and shown to be expressed during mouse embryogenesis. In addition, one of the most potent mesoderm-inducing factors, XTC-MIF, also appears to be structurally related to TGF-β [Rosa et al, Science 239:783-785 (1988); and Smith et al, Development 103:591-600 (1988)]. The TGF-β's themselves are capable of influencing a wide variety of differentiation processes, including adipogenesis, myogenesis, chondrogenesis, hematopoiesis, and epithelial cell differentiation [Massague, J., Cell 49:437-438 (1987)], and at least one TGF-β, namely TGF-β2, is capable of inducing mesoderm formation in frog embryos [Rosa et al, Science 239:783-785 (1988)].

The present invention relates to new members of the TGF-β superfamily, and to the nucleotide sequence encoding same. These new gene sequences and the encoded proteins, like other members of this superfamily, are likely play an important role in mediating developmental decisions related to cell differentiation.

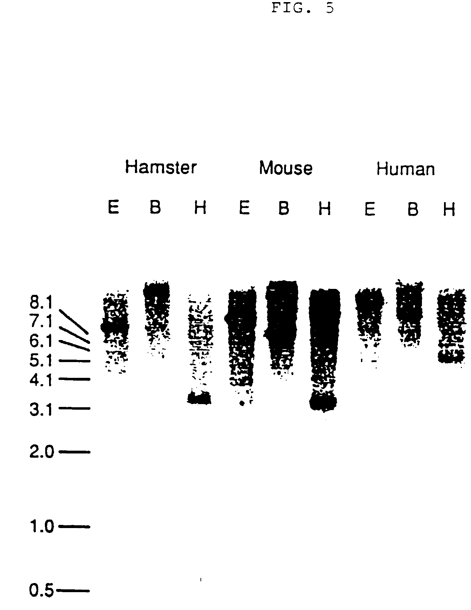

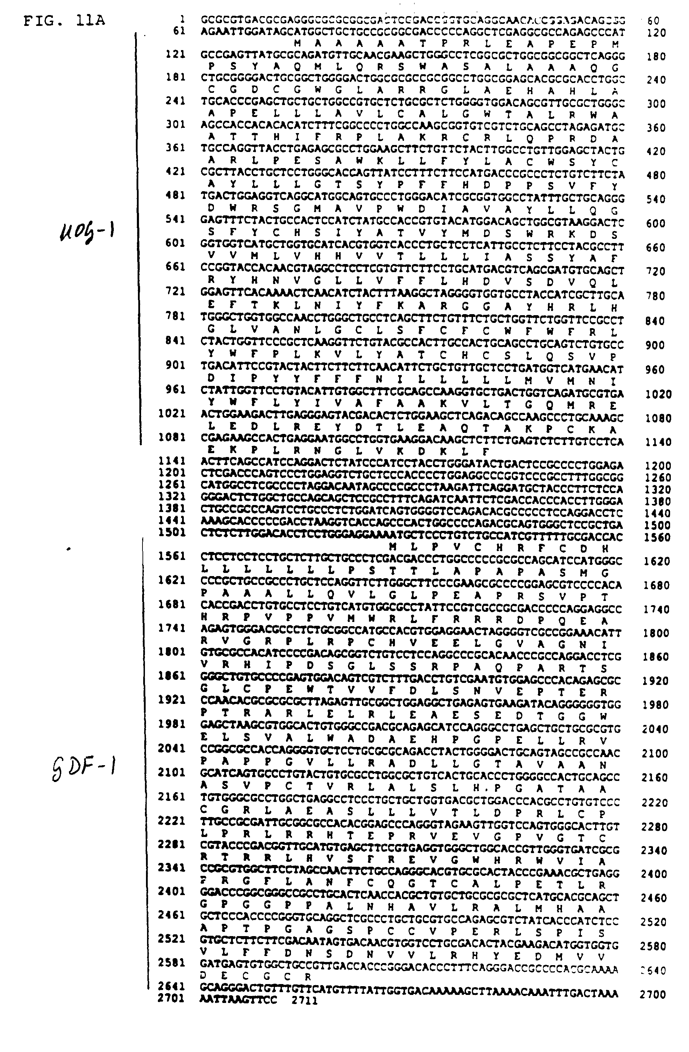

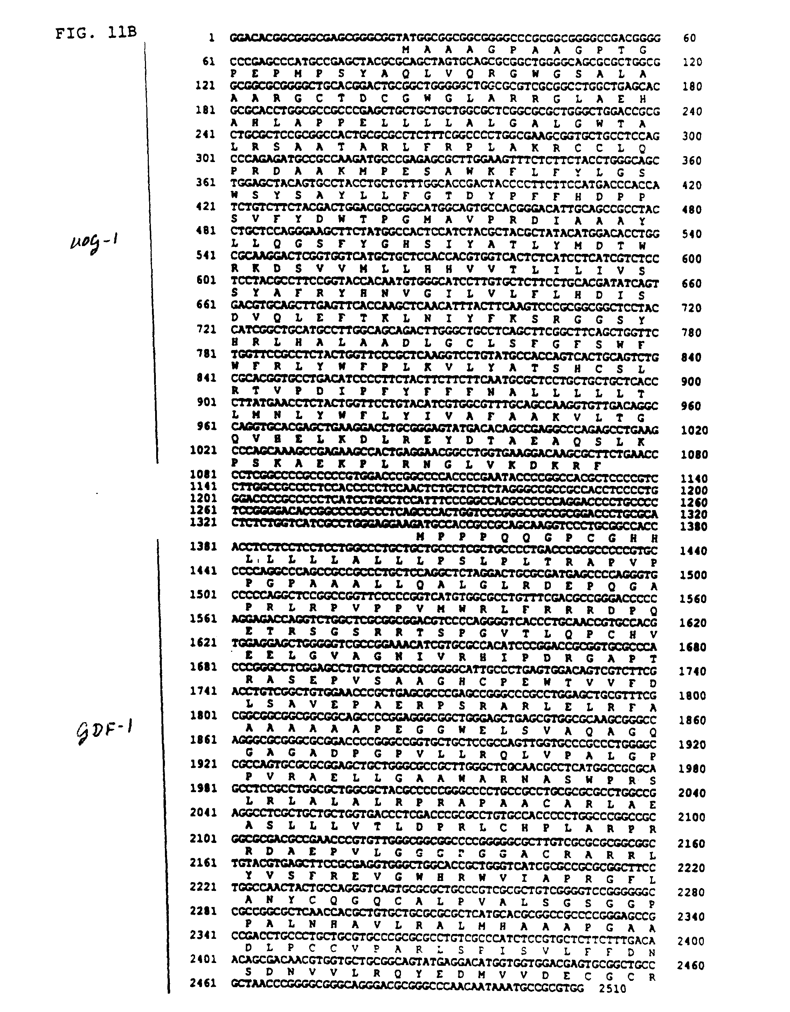

The present invention now provides a DNA segment encoding a mammalian GDF-1 protein being a mouse protein or a human protein of the sequence shown in Figures 2 and 11A for the mouse GDF-1 or in Figure 11B for human GDF-1 or encoding a mammalian UOG-1 protein being a mouse protein or human protein of the sequence shown in Figure 11A for the mouse UOG-1 or Figure 11B for the human UOG-1 or encoding a peptide including an epitope of any of the foregoing proteins, or a DNA fragment of at least 15 nucleotides of a said DNA segment or a complementary DNA to a said DNA segment.

The invention further provides a mammalian GDF-1 or UOG-1 protein (as defined above), substantially free of proteins with which it is naturally non-covalently associated, or a peptide including an epitope of a said protein.

The protein may be chemically synthesised and may be unglycosylated.

The invention includes a recombinant DNA molecule comprising:
i) a DNA segment as described above; and
ii) a vector.

The invention further includes a procaryotic or eucaryotic host cell stably transformed with a said recombinant DNA molecule.

The invention also includes a method of producing a recombinant GDF-1 or UOG-1 protein (as defined above), comprising culturing said host cell according to Claim 6 under conditions such that said segment is expressed and said GDF-1 or UOG-1 protein thereby produced, and isolating said GDF-1 or UOG-1 protein.

The invention also includes a monoclonal or polyclonal antibody immunologically reactive with such a protein or peptide.

The invention will be further explained and described with reference to the accompanying drawings in which:-

Figure 1 shows a Northern analysis of embryonic RNA. Two µg of twice-poly A-selected mRNA isolated from day 8.5 post-coitum (p.c.) mouse embryos were electrophoresed on formaldehyde gels, transferred to nitrocellulose, and probed with GDF-1 cDNA.

Figure 2 shows the sequence of GDF-1. The entire nucleotide sequence of GDF-1 derived from a single cDNA clone is shown with the predicted amino acid sequence below. The poly A tail is not shown.

Numbers indicate nucleotide position relative to the 5' end of the clone.

Figure 3 is a comparison of the predicted GDF-1 amino acid sequence with the amino acid sequences of previously-described members of the TGF-β superfamily.
(A): Alignment of the C-terminal amino acid sequences of GDF-1 (beginning at amino acid 236) with the corresponding regions of Xenopus Vg-1 [Weeks et al, Cell 51:861-867 (1987)], murine Vgr-1 [Lyons et al, Proc. Natl. Acad. Sci., USA, 86:4554-4558 (1989)], human BMP2a, 2b, and 3 [Wozney et al, Science 242, 1528-1534 (1988)] Drosophila DPP [Padgett et al, Nature 325:81-84 (1987)], human MIS [Cate et al, Cell 45:685-698 (1986)], human inhibin α, βA, and βB [Mason et al, Biochem Biophys Res Comm 135:957-964 (1986)], human TGF-β1 [Derynck et al, Nature 316:701-705 (1985)], human TGF-β2 [de Martin et al, EMBO J 6:3673-3677 (1987)], human TGF-β3 [ten Dijke et al, Proc. Natl. Acad. Sci., USA 85:4715-4719 (1988); and Derynck et al, EMBO J 7:3737-3743 (1988)], chicken TGF-β4 [Jakowlew et al, Mol Endocrinol 2:1186-1195 (1988)], and Xenopus TGF-85 [Kondaiah et al, J. Biol. Chem. 265:1089-1093 (1990)]. The 7 invariant cysteines are shaded. Dashes denote gaps introduced in order to maximize the alignment.
(B): Amino acid homologies among the different members of the superfamily. Numbers represent percent identities between each pair calculated from the first conserved cysteine to the C-terminus.
(C): Homology between GDF-1 and Vg-1 upstream of the presumed dibasic cleavage site. Two different regions are shown. A single gap of one amino acid has been introduced into the Vg-1 sequence in order to maximize the alignment. Numbers indicate amino acid positions in the respective proteins.

Figure 4 shows a sodium dodecyl sulfate-polyacrylamide gel electrophoresis (SDS-PAGE) of the in vitro translation product of GDF-1. Anti-sense (lane 1) or sense (lanes 2-13) RNA, transcribed and capped in vitro, was translated with a rabbit reticulocyte lysate in the presence of [³⁵S]methionine with (lanes 3, 5, 7, 9, 11, and 13) or without (lanes 1, 2, 4, 6, 8, and 12) added dog pancreas microsomes. Lanes: 2 and 3, translation products from a full-length GDF-1 template; 4 and 5, translation products from a deletion template lacking the putative signal sequence; 6 and 7, Endo-H treated translation products from a full-length GDF-1 template; 8 and 9, trypsin-treated translation products from a full-length GDF-1 template; 10 and 11, trypsin-treated translation products from a deletion template lacking the putative signal sequence; 12 and 13, translation products from a full-length GDF-1 template treated with trypsin in the presence of Triton X-100. Equal amounts of products prepared in a single translation reaction were used for lanes 2, 6, 8, and 12, for lanes 3, 7, 9, and 13, for lanes 4 and 10, and for lanes 5 and 11. Numbers at left indicate sizes of molecular weight standards. The 41K, 39.5K, and 38K positions were calculated relative to the mobilities of these standards.

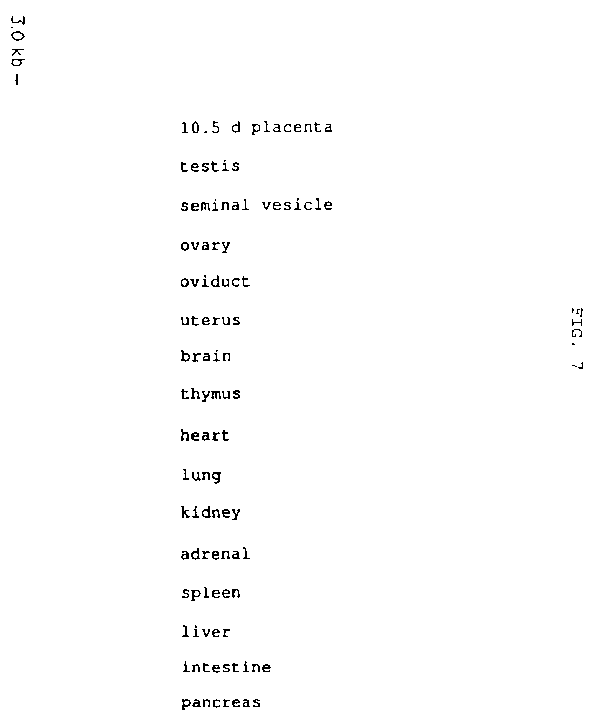

Figure 5 shows a genomic Southern analysis of GDF-1. Ten µg of genomic DNA isolated from CHO cells (hamster), BNL cells (mouse), or BeWo cells (human) were digested with Eco R1 (E), Bam HI (B), or Hind III (H), electrophoresed on a 1% agarose gel, transferred to nitrocellulose, and probed with GDF-1. Numbers at left indicate sizes (kb) of standards.

Figure 6 shows Northern analysis of embryonic RNA. Two µg of twice-poly A-selected mRNA isolated from mouse embryos at the indicated days of gestation were electrophoresed on formaldehyde gels, transferred to nitrocellulose, and probed with GDF-1 cDNA. The assignment of the sizes of the bands was based on the mobilities of RNA standards.

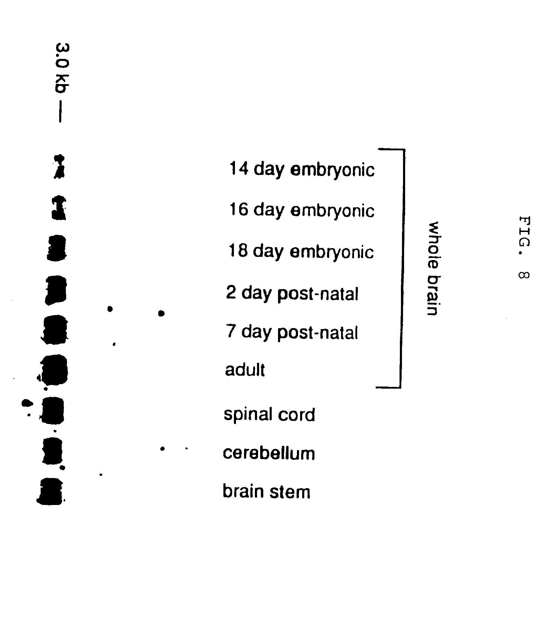

Figure 7 shows expression of GDF-1 in mouse tissues. Five µg of once-poly A-selected mRNA isolated from various mouse tissues were electrophoresed on formaldehyde gels, transferred to nitrocellulose, and probed with GDF-1 cDNA. The assignment of the size of the band was based on the mobilities of RNA standards.

Figure 8 shows expression of GDF-1 in the central nervous system. Two µg of twice poly A-selected mRNA isolated from fetal, neonatal, and adult brains, and from adult spinal cord, cerebellum, and brain stem were electrophoresed on formaldehyde gels, transferred to nitrocellulose, and probed with GDF-1 cDNA. The assignment of the size of the band was based on the mobilities of RNA standards.

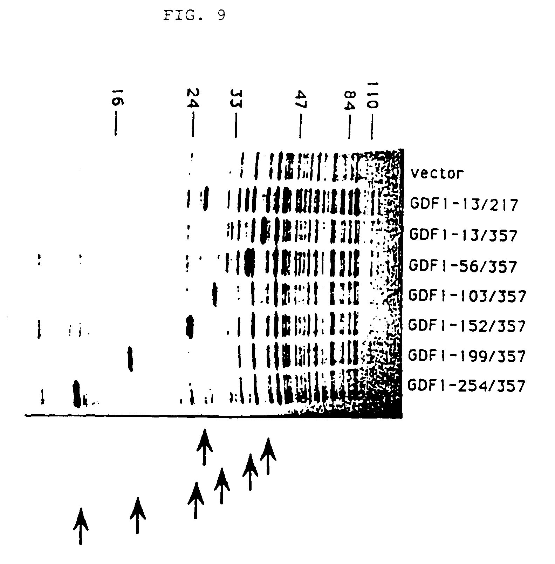

Figure 9 shows expression of GDF-1 in bacteria. Portions of GDF-1 cDNA were cloned into the pET3 vector and transformed into BL21 (DE3) cells. Total bacterial extracts were electrophoresed on 15% SDS polyacrylamide gels and stained with Coomassie blue. The numbers at top indicate the first/last amino acid of GDF-1 contained in each construct. Numbers at left indicate sizes of molecular weight standards. Arrows at right indicate the positions of the bands representing GDF-1.

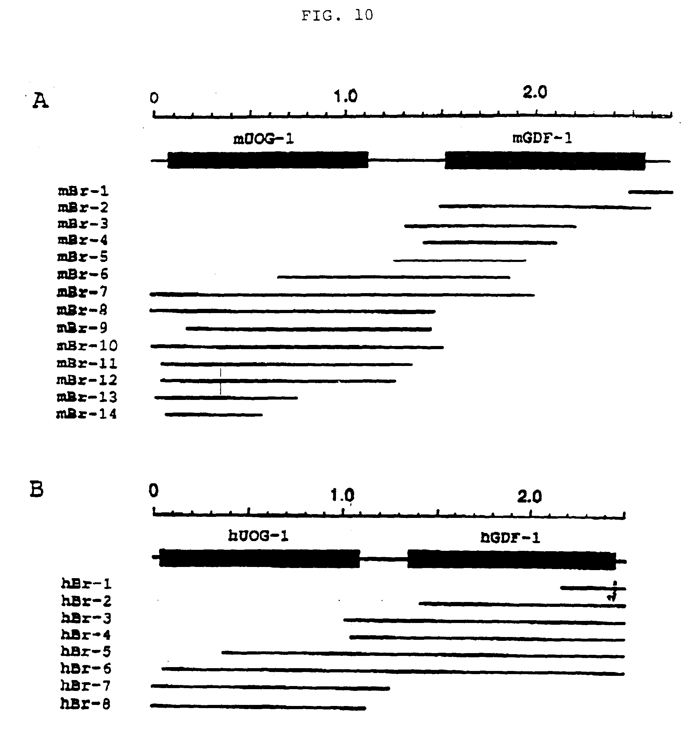

Figure 10 shows a schematic representation of clones isolated from brain cDNA libraries. (A) oligo dT-primed and random hexanucleotide-primed murine brain cDNA libraries were prepared in the lambda ZAP II vector (Stratagene) using the RNase H procedure [Okayama et al, Mol. Cell. Biol. 2:161 (1982); Gubler et al, Gene 25:263 (1983)] according to the instructions provided by Stratagene and Amersham, respectively. Two separate oligo dT-primed libraries of 0.7 million (library 1) and 2 million (library 2) recombinant phage and a random-primed library of 1.3 million (library 3) recombinant phage were obtained from 2 µg of twice poly A-selected adult brain mRNA per library. Library 1 was amplified once prior to screening, whereas libraries 2 and 3 were screened unamplified. Hybridizations were carried out in 1M NaCl, 50 mM sodium phosphate (pH 6.5), 2 mM EDTA, 0.5% SDS, and 10 X Denhardt's at 65°C. The final wash was carried out in 0.5 X SSC at 68°C. (B) human adult cerebellum and human fetal brain (17 to 18 week abortus) cDNA libraries were obtained from Stratagene. Hybridizations were carried out as for Figure 10(A) except that the final wash was carried out in 2 X SSC at 65°C. Numbers above the scales represent kb. The locations of the UOG-1 and GDF-1 open reading frames are shown by the solid and stippled boxes, respectively. All clones were oriented and aligned by determining the sequences at both ends.

Figure 11 shows the nucleotide sequences of murine and human cDNA's encoding UOG-1 and GDF-1. DNA sequences of both strands of murine (A) and human (B) cDNA clones were determined with the dideoxy chain termination method [Sanger et al, Proc. Natl. Acad. Sci., USA 74:5463 (1977)] using the exonuclease III/S1 nuclease strategy [Henikoff, Gene 28:351 (1984)]. The specific clones sequenced to assemble the complete sequences shown are described in the Examples below. Numbers indicate nucleotide position relative to the 5' end. The predicted amino acid sequences of UOG-1 and GDF-1 are shown below.

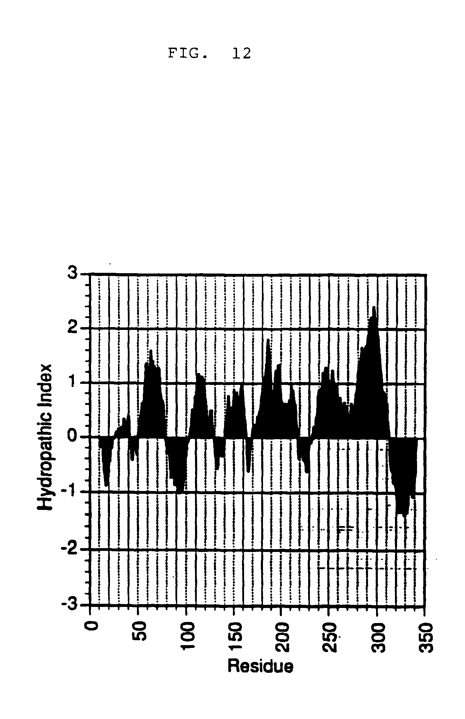

Figure 12 shows the hydropathicity profile of mUOG-1. Average hydrophobicity values were calculated using the method of J. Kyte and R.F. Doolittle, J. Mol. Biol. 157:105 (1982). Positive numbers indicated increasing hydrophobicity.

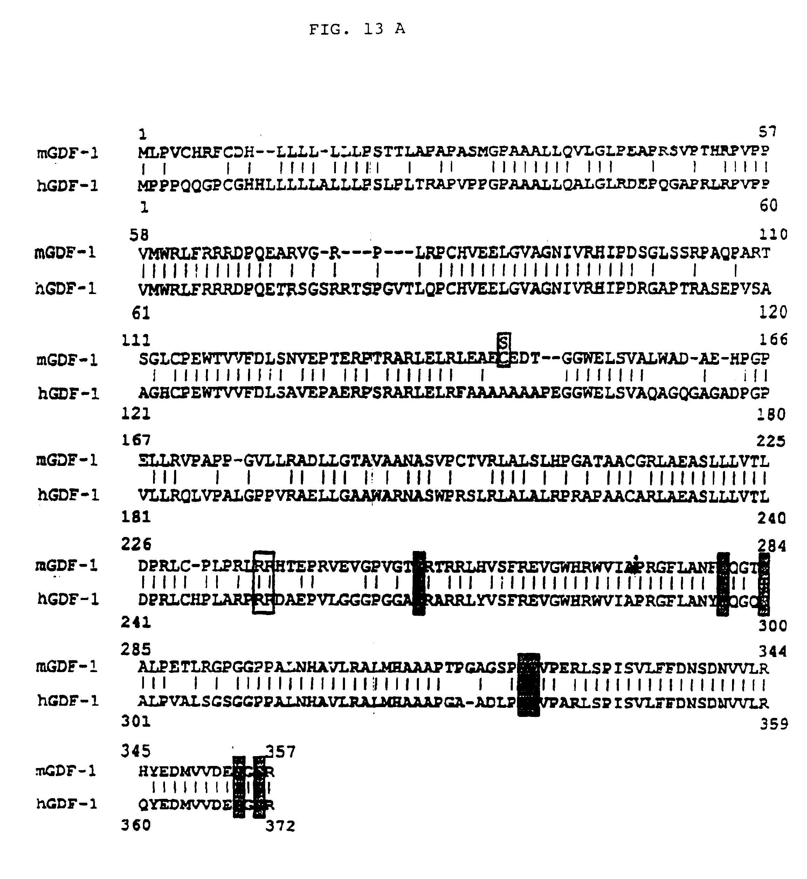

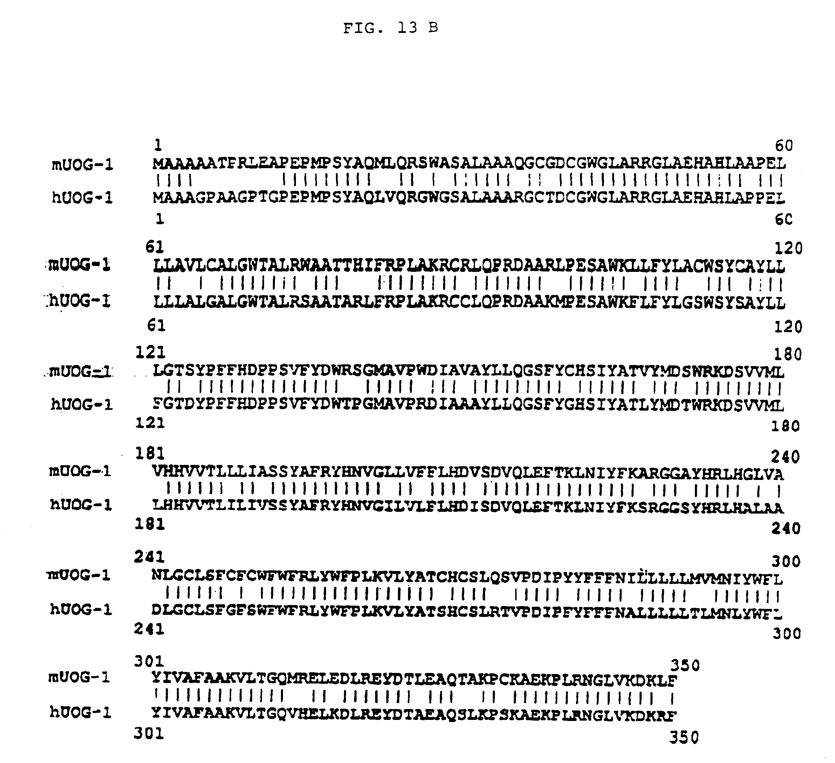

Figure 13 shows the alignment of murine and human sequences. Amino acid alignment of mGDG-1 with hGDF-1 (A) or mUOG-1 with hUOG-1 (B) were carried out using the SEQHP local homology program. Numbers indicate amino acid number relative to the N-terminus of each protein. Dashes denote gaps introduced in order to maximize the alignment. The 7 invariant cysteines in the GDF-1 sequences are shaded. The predicted dibasic cleavage sites are boxed. The box at position 145 in the mGDF-1 sequence shows the alternative amino acids at this position for GDF-1a (cysteine) or GDF-1b (serine). (C) DIAGON plot of murine and human nucleotide sequences was carried out with a window of 20 and stringency of 14. The locations of the UOG-1 and GDF-1 open reading frames are shown by the solid and stippled boxes, respectively. Numbers indicate nucleotide position in thousands.

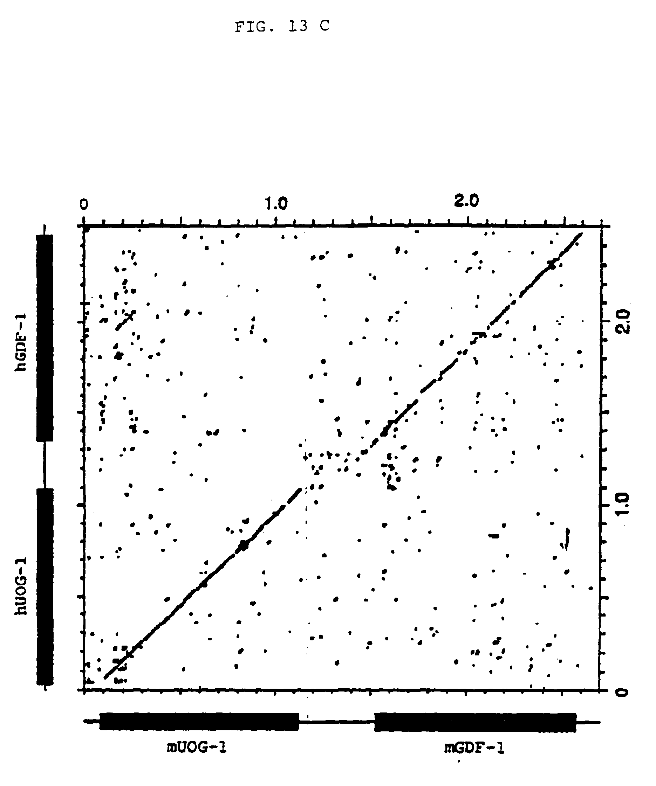

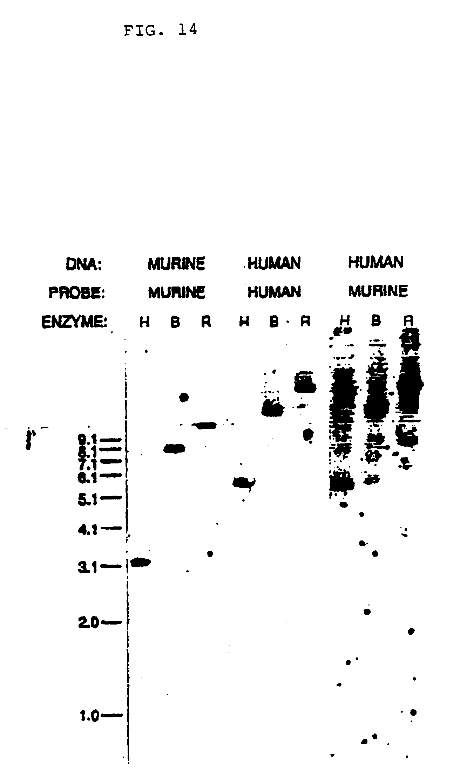

Figure 14 shows a genomic Southern analysis of GDF-1. Ten micrograms of genomic DNA isolated from BNL cells (murine) or BeWo cells (human) were digested with Hind III (H), Bam HI (B), or Eco RI (R), electrophoresed on 1% agarose gels, transferred to nitrocellulose, and probed with the entire murine or human GDF-1 coding sequences as described in the legend to Figure 10. Filters hybridized with probes from the homologous species were washed in 0.2 X SSC at 68°C, whereas the filter containing human DNA probed with mGDF-1 was washed in 2 X SSC at 68°C. Numbers at left indicate sizes of standards in kb.

The present invention includes a DNA segment that encodes the entire amino acid sequence given in Figure 2 (the specific DNA segment given in Figure 2 being only one such example), or any unique portion thereof. A "unique portion" as used herein consists of at least five (or six) amino acids or, correspondingly, at least 15 (or 18) nucleotides. The present invention further relates to a recombinant DNA molecule comprising the above DNA segment and to host cells transformed therewith. DNA segments to which the invention relates also include those encoding substantially the same protein as shown in Figure 2, including, for example, allelic variations of the amino acid sequence of Figure 2. The invention also relates to nucleotide fragments complementary to such DNA segments. Unique portions of the DNA segment, or complementary fragments, can be used as probes for detecting the presence of respective complementary strands in DNA (or RNA) samples.

The present invention further relates to GDF-1 substantially free of proteins with which it is normally non-covalently associated, or a unique peptide fragment of that protein. One skilled in the art can purify the GDF-1 using standard methodologies for protein purification. The GDF-1 protein, or peptide fragments thereof, to which the invention relates also include those which have been chemically synthesized using known methods. One skilled in the art will appreciate that multiple copies of the GDF-1 gene may exist. Each of the encoded proteins will likely carry out functions similar to or identical to the protein of Figure 2. Therefore, the term GDF-1 applies to these forms as well.

GDF-1 has potential N-linked glycosylation sites. Accordingly, one skilled in the art, without undue experimentation, can modify, partially remove or completely remove, the natural glycosyl groups from the GDF-1 protein using standard methodologies. Therefore, the proteins and peptides of the present invention may be glycosylated or unglycosylated.

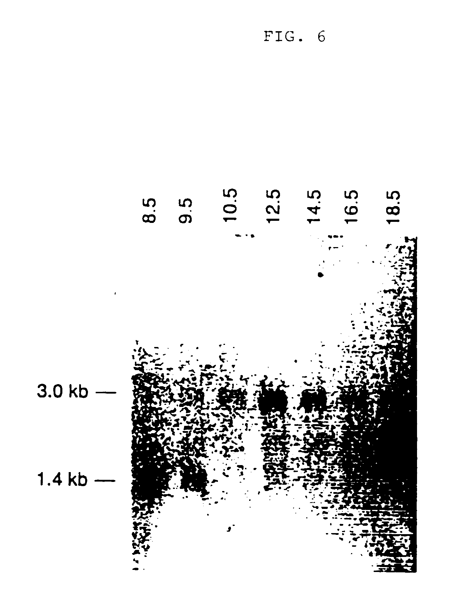

The present invention also relates to recombinantly produced GDF-1 having the amino acid sequence given in Figure 3 including allelic, variations thereof. The recombinantly produced protein may be unglycosylated or glycosylated (the glycosylation pattern may differ from that of the naturally occurring protein). The present invention further relates to recombinantly produced unique peptide fragments of GDF-1.

The present invention also relates to a recombinant DNA molecule and a to host cell transformed therewith. Using standard methodologies, well known in the art, a recombinant DNA molecule comprising a vector and a DNA segment encoding GDF-1, or a unique portion thereof, can be constructed. Vectors suitable for use in the present invention include, but are not limited to, baculovirus-derived vectors for expression in insect cells [Pennock et al, Mol. Cell. Biol. 4:399-406 (1984)], the T7-based expression vector for expression in bacteria [Rosenberg et al, Gene 56:125-135 (1987)] and the pMSXND expression vector for expression in mammalian cells [Lee and Nathans, J. Biol. Chem. 263:3521-3527 (1988)]. The DNA segment can be present in the vector operably linked to regulatory elements, for example, a promoter (e.g., polyhedrin, T7 or metallothionein I (Mt-I) promoters). The recombinant DNA molecule is suitable for transforming prokaryotic or eukaryotic cells.

The recombinant DNA molecule of the invention can be introduced into appropriate host cells by one skilled in the art using methods well known in the art. Suitable host cells include prokaryotic cells, such as bacteria, lower eukaryotic cells, such as yeast, and higher eukaryotic cells, such as mammalian cells and insect cells.

The proteins and unique peptides of the invention can be used as antigens to generate GDF-1 specific antibodies using methods known in the art. Therefore, the invention also relates to monoclonal and polyclonal GDF-1 specific-antibodies.

The TGF-β superfamily encompasses a group of proteins affecting a wide range of differentiation processes. The structural homology between GDF-1 and the known members of the TGF-β superfamily and the pattern of expression GDF-1 during embryogenesis indicate that GDF-1 is a new member of this family of growth and differentiation factors. Based on the known properties of the other members of the this superfamily, GDF-1 can be expected to possess biological properties of diagnostic and/or therapeutic benefit in a clinical setting.

For example, one potential use for GDF-1 as a diagnostic tool is as a specific marker for the presence of tumors arising from cell types that normally express GDF-1. The availability of such markers would be invaluable for identifying primary and metastatic neoplasms of unknown origin or for monitoring the response of an identified neoplasm to a particular therapeutic regimen. In this regard, one member of this superfamily, namely, inhibin, has been shown to be useful as a marker for certain ovarian tumors [Lappohn et al, N. Engl. J. Med. 321:790 (1989)].

A second potential diagnostic use for GDF-1 is as an indicator for the presence of developmental anomalies in prenatal screens for potential birth defects. For example, abnormally high serum or amniotic fluids levels of GDF-1 may indicate the presence of structural defects in the developing fetus. Indeed, another embryonic marker, namely, alpha fetoprotein, is currently used routinely in prenatal screens for neural tube defects [Haddow and Macri, JAMA 242:515 (1979)]. Conversely, abnormally low levels of GDF-1 may indicate the presence of developmental anomalies directly related to the tissues normally expressing GDF-1.

A third potential diagnostic use for GDF-1 is in prenatal screens for genetic diseases that either directly correlate with the expression or function of GDF-1 or are closely linked to the GDF-1 gene. Other potential diagnostic uses will become evident upon further characterization of the expression and function of GDF-1.

Potential uses for GDF-1 as a therapeutic tool are also suggested by the known biological activities of the other members of this superfamily. For example, since some of these proteins act as cell-specific growth inhibitors, one potential therapeutic use for GDF-1 is as an anti-cancer drug to inhibit the growth of tumors derived from cell types that are normally responsive to GDF-1. Indeed, one member of this superfamily, namely, Mullerian inhibiting substance, has been shown to be cytotoxic for human ovarian and endometrial tumor cells either grown in culture [Donahoe et al, Science 205:913 (1979); Fuller et al, J. Clin. Endocrinol. Metab. 54:1051 (1982)] or when transplanted into nude mice [Donahoe et al, Ann. Surg. 194:472 (1981); Fuller et al, Gynecol. Oncol. 22:135 (1984)].

Conversely, if GDF-1 functions as a growth-stimulatory factor for specific cell types, other potential therapeutic uses will be apparent. For example, one member of this superfamily, namely, activin, has been shown to function as a nerve cell survival molecule [Schubert et al, Nature 344:868 (1990)]. If GDF-1 possesses a similar activity, as is indicated by its specific expression in the central nervous system (see below), GDF-1 will likely prove useful in vitro for maintaining neuronal cultures for eventual transplantation or in vivo for rescuing neurons following axonal injury or in disease states leading to neuronal degeneration. Alternatively, if the target cells for GDF-1 in the nervous system are the support cells, GDF-1 will likely prove to be of therapeutic benefit in the treatment of disease processes leading to demyelination.

Many of the members of this superfamily, including GDF-1, are also likely to be clinically useful for tissue repair and remodeling. For example, the remarkable capacity of the bone morphogenetic proteins to induce new bone growth [Urist et al, Science 220:680 (1983)] has suggested their utility for the treatment of bone defects caused by trauma, surgery, or degenerative diseases like osteoporosis. Indeed, the bone morphogenetic proteins have already been tested in vivo in the treatment of fractures and other skeletal defects [Glowacki et al, Lancet i:959 (1981); Ferguson et al, Clin. Orthoped. Relat. Res. 227:265 (1988); Johnson et al, Clin. Orthoped. Relat. Res. 230:257 (1988)].

A determination of the specific clinical settings in which GDF-1 will be used as a diagnostic or as a therapeutic tool await further characterization of the expression patterns and biological properties of GDF-1 both under normal physiological conditions and during disease states. Based on the wide diversity of settings in which other members of this superfamily may be used for clinical benefit, it is likely that GDF-1 and/or antibodies directed against GDF-1, will also prove to be enormously powerful clinical tools. Potential uses for GDF-1 will almost certainly include but not be restricted to the types of clinical settings described above. Moreover, as methods for improving the delivery of drugs to specific tissues or to specific cells become available, other uses for molecules like GDF-1 will become evident.

The following non-limiting Examples are provided to aid in the understanding of the present invention. In addition, data presented in the Examples (see, particularly, Examples 7 and 8) make possible a comparison of murine and human sequences derived from brain cDNA clones. The comparison reveals high conservation of two non-overlapping open reading frames. While the downstream open reading frame encodes GDF-1, the upstream open reading frame encodes a protein, designated UOG-1, containing multiple putative membrane-spanning domains. The data indicate that this mRNA gives rise to two different proteins. The bi-cistronic organization of UOG-1 and GDF-1 is unusual for eucaryotic mRNA's. Polycistronic mRNA's in procaryotes, however, often encode proteins carrying out related biological functions. Accordingly, UOG-1 and GDF-1 may functionally interact. The presence of multiple putative membrane scanning domains in UOG-1 indicates it may be a receptor, perhaps for GDF-1.

### EXAMPLES

The following technical comments relate to the specific Examples that follow:
Construction and screening of an 8.5 day embryonic cDNA library: All embryonic materials were obtained from random matings of CD-1 mice (Charles River). Mice were maintained according to the NIH guidelines for care and maintenance of experimental animals. The day on which the vaginal plug was noted was designated as day 0.5 p.c. Embryos were dissected out from the uterus, freed of all extra-embryonic membranes, and frozen rapidly. Total RNA was prepared by homogenization in guanidinium thiocyanate buffer and centrifugation of the lysate through a cesium chloride cushion [Chirgwin et al, Biochemistry 18:5294-5299 (1979)]. Poly A-containing RNA was obtained by twice-selecting with oligo-dT cellulose [Aviv, H., Proc. Natl. Acad. Sci. USA 69:1408-1412 (1972)]. A cDNA library was constructed in the lambda ZAP II vector using the RNase H method [Okayama et al, Mol. Cell Biol. 2:161-170 (1982); and Gubler et al, Gene 25:263-269 (1983)] according to the instructions provided by Stratagene. Recombinant plaques (3.2 million) were obtained from 2 µg of starting RNA. The library was screened with the oligonucleotide 5'-GCAGCCACACTCCTCCACCACCATGTT-3' (corresponding to the amino acid sequence NMVVEECGC) which had been end-labeled using polynucleotide kinase. Hybridization was carried out in 6X SSC, 1X Denhardt's, 0.05% sodium pyrophosphate, 100 µg/ml yeast tRNA at 50°C. Filters were washed in 6X SSC, 0.05% sodium pyrophosphate at 60°C.
DNA sequencing and blot hybridizations: DNA sequencing of both strands was carried out with the dideoxy chain termination method [Sanger et al, Proc. Natl. Acad. Sci., USA 74:5463-5467 (1977)] using the exonuclease III/S1 nuclease strategy [Henikoff S., Gene 28:351-359 (1984)].

For Northern analysis, RNA was electrophoresed on formaldehyde gels [(Lehrach et al, Biochemistry 16:4743-4751 (1977); and Goldberg, D.A., Proc. Natl. Acad. Sci., USA 77:5794-5798 (1980)], transferred to nitrocellulose, and hybridized in 50% formamide, 5X SSC, 4X Denhardt's, 0.1% SDS, 0.1% sodium pyrophosphate, 100 µg/ml salmon DNA at 50°C. Filters were washed first in 2X SSC, 0.1% SDS, 0.1% sodium pyrophosphate, then in 0.1X SSC, 0.1% SDS at 50°C.

For Southern analysis, DNA was electrophoresed on 1% agarose gels, transferred to nitrocellulose, and hybridized in 1M NaCl, 50 mM sodium phosphate, pH 6.5, 2 mM EDTA, 0.5% SDS, 10X Denhardt's at 65°C. The final wash was carried out in 2X SSC at 68°C.
In vitro translation experiments: The full-length 1387 bp GDF-1 cDNA or a deletion mutant lacking the first 251 nucleotides was subcloned into the Bluescript vector (Stratagene), and sense or anti-sense RNA was transcribed in vitro from the T3 or T7 promoters [Golomb et al, J. Virol 21:743-752 (1977); and McAllister et al, Nucl. Acids Res. 8:4821-4837 (1980)] in the presence of cap analog, as described by Stratagene. In vitro translations were carried out by incubating 0.5 µg RNA, 17.5 µl rabbit reticulocyte lysate (Promega), 20 µM cold amino acid mixture (Promega), and 20 µCi [³⁵S]methionine (New England Nuclear) in the presence or absence of 10 equivalents of dog pancreas microsomes (Promega) for 60 minutes at 30°C. Endoglycosidase digestions were carried out by diluting the translation reaction 1:30 with 100 mM sodium acetate pH 5.5, 0.1% SDS, 17 mU/ml endoglycosidase H (Boehringer-Mannheim). Protease digestions were carried out by diluting the translation reaction 1:20 with PBS, 1 mg/ml trypsin (Boehringer-Mannheim) in the presence or absence of 0.1% Triton X-100. All digestions were carried out for 3 hours at 37°C. Translation products were analyzed by electrophoresis on 10% SDS polyacrylamide gels [Laemmli, U.K., Nature 227:680-685 (1970)] followed by fluorography with Enhance (New England Nuclear).

### Example 1

### Cloning and Nucleotide Sequence of GDG-1

To identify new members of the TGF-β superfamily that may be important for mouse embryogenesis, a cDNA library was constructed in lambda Zap II using poly A-selected RNA from whole embryos isolated at day 8.5 p.c. As indicated above, the library was screened with oligonucleotides selected on the basis of the predicted amino acid sequences of conserved regions among members of the superfamily. Among 600,000 recombinant phage screened, the oligonucleotide hybridized to 3 clones. Sequence analysis revealed that the 3 cDNA clones were likely to represent mRNA's derived from the same gene, which was designated GDF-1.

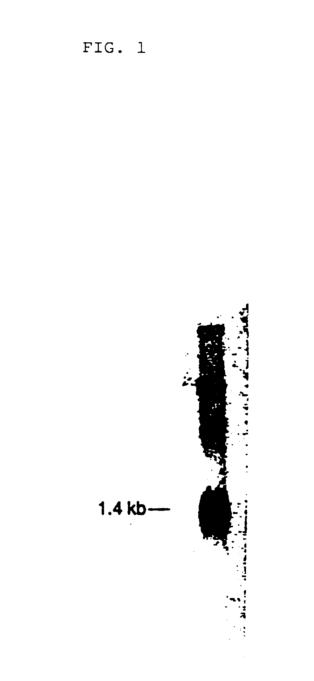

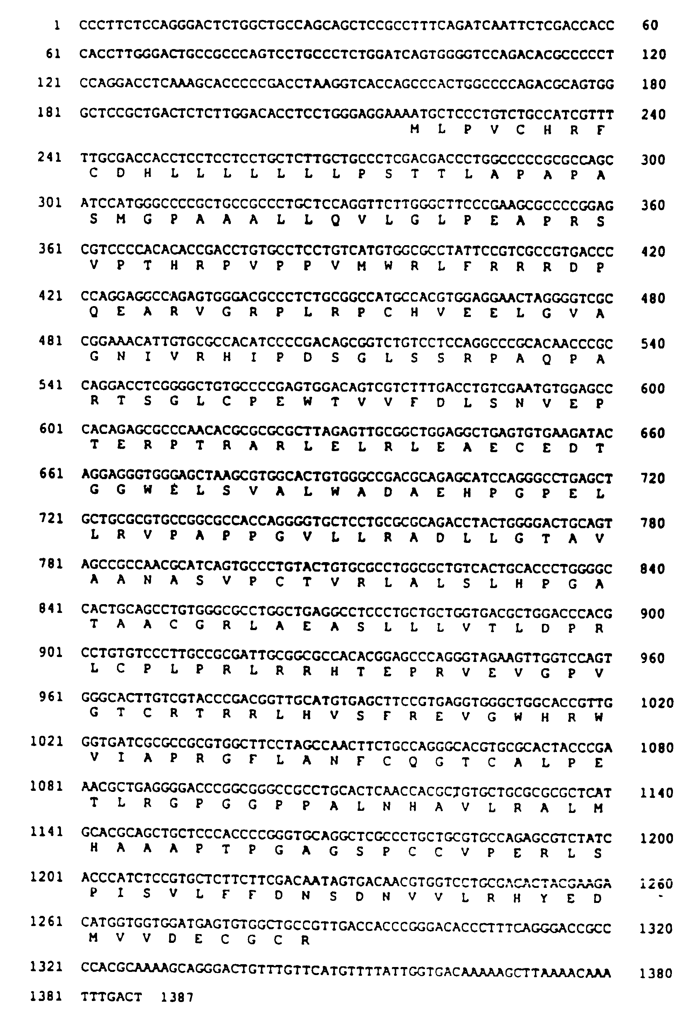

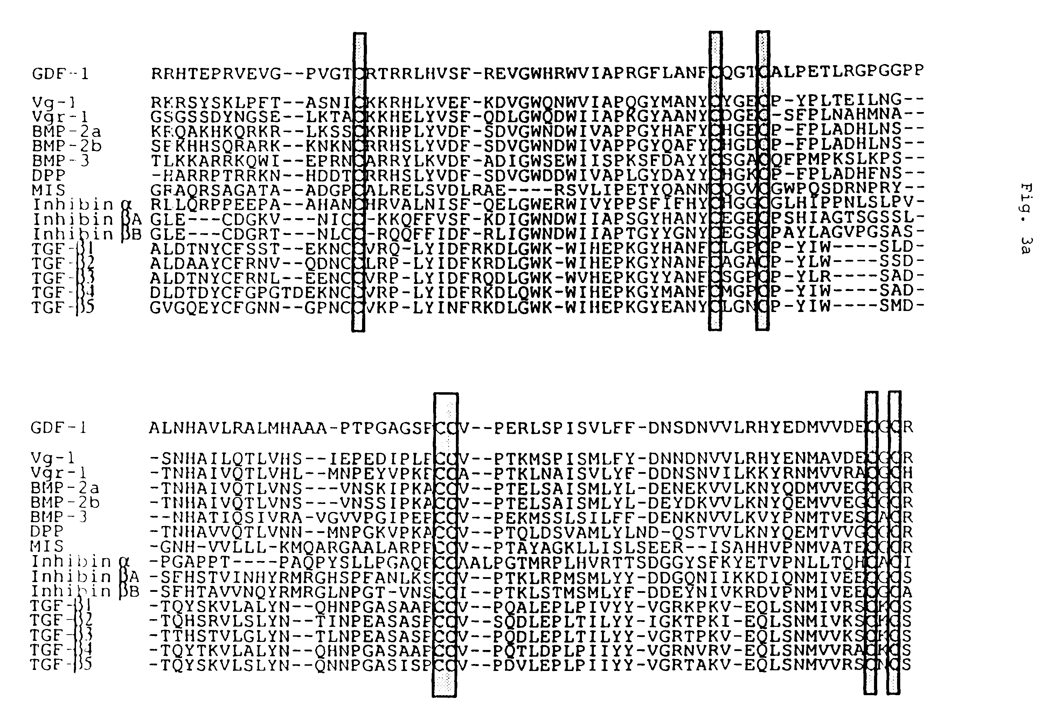

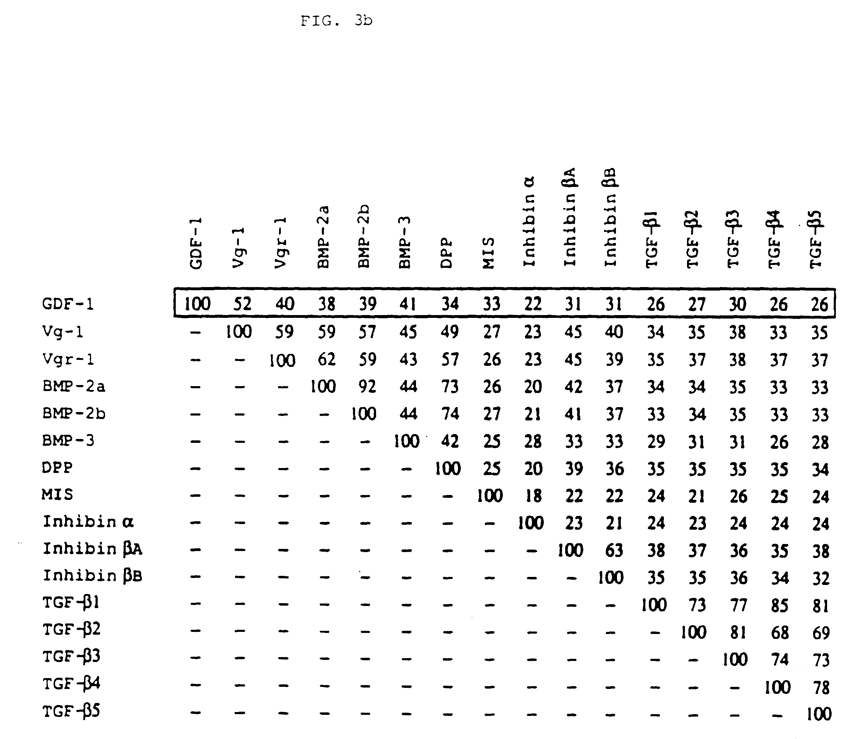

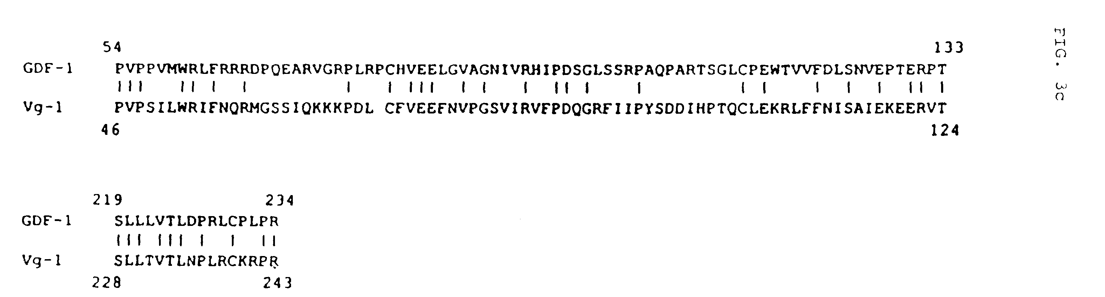

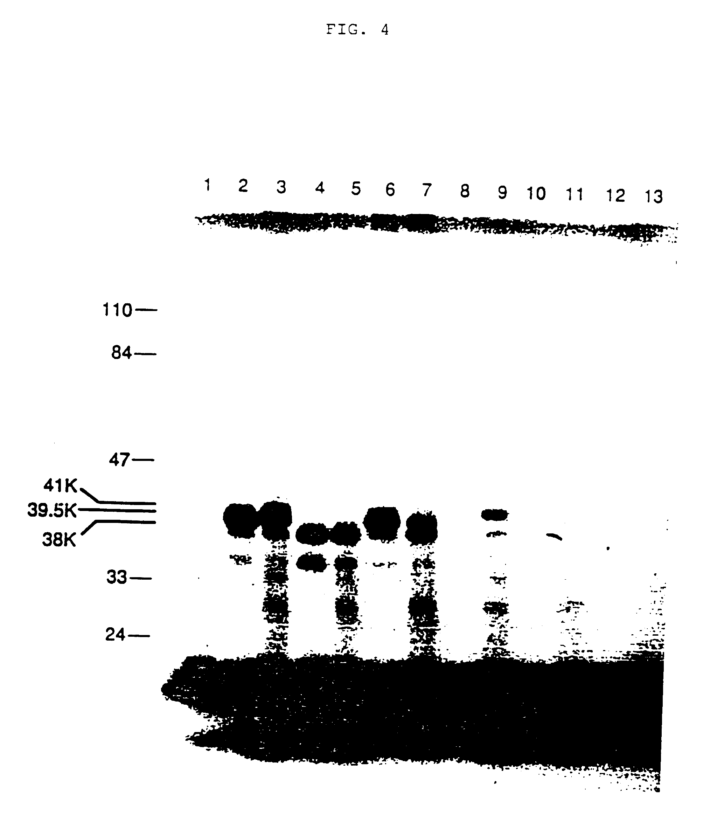

Northern analysis of day 8.5 embryonic RNA using the GDF-1 probe detected a single predominant mRNA species of approximately 1.4 kb in length (Figure 1). Because the original 3 cDNA isolates were all smaller than 1.4 kb, portions of the longest clone were used to re-screen the cDNA library to isolate a full-length clone. Hybridizing recombinant phage were seen at a frequency of approximately 1 per 200,000.

The entire nucleotide sequence of the longest cDNA clone obtained encoding GDF-1 is shown in Figure 2. The 1387 bp sequence contains a single long open reading frame beginning with an initiating ATG at nucleotide 217 and potentially encoding a protein 357 amino acids with a molecular weight of 38,600. Upstream of the putative initiating ATG are two in-frame stop codons and no additional ATG's. Nucleotides 1259 to 1285 show a 25/27 match with the complement of the oligonucleotide selected for the original screening. The 3' end of the clone does not contain the canonical AAUAAA polyadenylation signal. Sequence analysis at the 3' end of 4 independent cDNA clones (all differing at their 5' ends) showed that 2 clones terminated at the same nucleotide, and the other 2 clones terminated at a site 7 nucleotides further downstream (these clones contained an additional AAAAATT sequence at the 3' end).

Two cDNA clones isolated during this screening process showed slight variations in their sequence from that shown in Figure 2. In a limited segment from which the nucleotide sequence was determined, these 2 clones each showed 2 nucleotide changes, one resulting in a cysteine to serine substitution at amino acid 145 and the second representing a third position change that did not alter the amino acid sequence. These differences are unlikely to be cloning artifacts since they were found in independently-isolated clones. These changes may represent allelic differences or they may indicate the presence of multiple GDF-1 genes.

The predicted amino acid sequence identified GDF-1 as a new member of the TGF-β superfamily. A comparison of the C-terminal 122 amino acids with those of the other members of this family is shown in Figure 3a. The predicted GDF-1 sequence contains all of the invariant amino acids present in the other family members, including the 7 cysteine residues with their characteristic spacing, as well as many of the other highly conserved amino acids. In addition, like other family members, the C-terminal portion of the predicted GDF-1 polypeptide is preceded by a pair of arginine residues at positions 236-237, potentially representing a site for proteolytic processing.

Figure 3b shows a tabulation of the percentages of identical residues between GDF-1 and the other members of the TGF-β family in the region starting with the first conserved cysteine and extending to the C-terminus. GDF-1 is most homologous to Vg-1 (52%) and least homologous to inhibin-a (22%) and the TGF-B's (26-30%). Two lines of reasoning indicate that GDF-1 is not the murine homolog of Vg-1. First, GDF-1 is less homologous to Vg-1 than are Vgr-1 (59%), BMP-2a(59%), and BMP-2b (57%). Second, GDF-1 does not show extensive homology with Vg-1 outside of the C-terminal portion, and it is known that other members of this family are highly conserved across species throughout the entire length of the protein [Cate et al, Cell 45:685-698 (1986); Mason et al, Nature 318:659-663 (1985); Forage et al, Proc. Natl. Acad. Sci., USA 83:3091-3095 (1986); Derynck et al, Nature 316:701-705 (1985); Mason et al, Biochem. Biophys. Res. Comm. 135:957-964 (1986); and Derynck et al, J. Biol. Chem. 261:4377-4379 (1986)]. However, GDF-1 and Vg-1 do share two regions of limited homology N-terminal to the presumed dibasic cleavage site, as shown in Figure 3c.

### Example 2

### In vitro translation of GDF-1 RNA

The predicted GDF-1 sequence is also noteworthy for the presence of a core of hydrophobic amino acids at the N-terminus, potentially representing a signal sequence, as well as for the presence of a potential N-glycosylation site at amino acid 191. To determine whether these sequences are functional and to confirm that translation initiates as predicted at the first ATG, in vitro translation experiments were carried out using a rabbit reticulocyte lysate.

As shown in Figure 4 (lane 2), translation of full-length sense GDF-1 RNA, transcribed and capped in vitro, resulted in a major protein species with a molecular weight of 39.5K, which agreed well with the predicted molecular weight of 38.6K for the translation product initiating at the most upstream ATG; no such band was seen with translation of anti-sense GDF-1 RNA (lane 1).

Support for translation initiation at the most upstream ATG came from a starting DNA template containing a deletion at the 5' end extending past the first ATG codon resulting in a slightly smaller translation product (lane 4), indicating that translation in this case had initiated at the next ATG codon (nucleotide 305). When full-length GDF-1 RNA was translated in the presence of dog pancreas microsomes, some of the translated product migrated slower than the full-length product (lane 3). This slower migrating species (41K) could be converted to a 38K form by treatment with endoglycosidase H (lane 7), consistent with the 41K and 38K species representing the glycosylated and deglycosylated forms, respectively, of the GDF-1 prptein lacking a signal peptide. Furthermore, the 41K species (unlike the unprocessed 39.5K species) was resistant to treatment with trypsin in the absence (lane 9) but not in the presence (lane 3) of detergent, suggesting that the 41K species was protected from cleavage by its presence within the microsomes.

In contrast, parallel experiments carried out with protein translated from a deletion template lacking the signal sequence showed no shift to a high molecular weight species in the presence of microsomes (lane 5) and no protection from cleavage by trypsin (lane 11). Taken together, these data indicate that GDF-1 is a secreted glycoprotein like many of the other members of this superfamily.

### Example 3

### Southern blot analysis

To determine whether GDF-1 is a single-copy gene, Southern blot analysis was carried out using mouse genomic DNA as described above. As shown in Figure 5, the GDF-1 probe detected a single predominant band in 3 different digests of mouse DNA. However, even at high stringency, additional weakly hybridizing bands were detected. These minor bands are not likely to represent the products of partial digestion because many of these bands were smaller than the predominant band, and the intensities of these minor bands relative to the major band could be enhanced by reducing the stringency of the washing conditions.

Southern analysis was also extended to DNA isolated from other species. Even at high stringency, the GDF-1 probe detected a single predominant band in both hamster and human DNA (see Figure 5), indicating that GDF-1 is highly conserved across species. Moreover, as was seen with mouse DNA, additional minor bands could be detected in both human and hamster DNA at relatively high stringency.

### Example 4

### Expression of GDF-1

To determine the temporal pattern of expression of GDF-1 during embryogenesis, Northern analysis was carried out using poly A-selected RNA prepared from whole embryos isolated at days 8.5, 9.5, 10.5, 12.5, 14.5, 16.5, and 18.5. The GDF-1 probe detected two mRNA species showing distinct expression patterns (Figure 6). One mRNA species, 1.4 kb in length, was detected in embryos at days 8.5 and 9.5 but not in later stage embryos. The second mRNA species, 3.0 kb in length, appeared at day 9.5 and persisted throughout embryogenesis. The 1.4 kb species is likely to correspond to the GDF-1 cDNA sequence shown in Figure 2 since only the 1.4 kb species could be detected in day 8.5 embryos.

Northern analysis was also carried out using poly A-selected RNA prepared from a variety of adult tissues. As shown in Figure 7, the GDF-1 probe detected a 3.0 kb mRNA species expressed almost exclusively in the brain. Significantly lower, though detectable levels, were seen in the adrenal gland, ovary, and oviduct. No band corresponding to 1.4 kb was detected in any of these adult tissues. To further analyze the expression of the 3.0 kb mRNA in the brain, poly A-selected RNA was prepared from brains isolated at various developmental stages as well as from various subcompartments of the adult central nervous system. As shown in Figure 8, the GDF-1 probe detected a 3.0 kb mRNA species in embryonic and neonatal brains with the levels gradually increasing during brain development. Moreover, the 3.0 kb mRNA was also present at high levels in the spinal cord, cerebellum, and brain stem, suggesting that the expression of the 3.0 kb species may be widespread in the central nervous system. In contrast, the 1.4 kb mRNA species was not detected in any of these samples.

In summary, the GDF-1 probe identified two mRNA species displaying distinct expression patterns. The 1.4 kb species, which corresponds to the cDNA sequence shown in Figure 2, was detected in embryos at day 8.5 and day 9.5 but not in later stage embryos or in any of the adult tissues tested. The 3.0 kb species appeared at day 9.5, persisted throughout embryonic development, and was present almost exclusively in the central nervous system of adult animals. The 3.0 kb and the 1.4 kb species may be derived from two different genes or they may represent alternatively initiated or processed transcripts, both derived from the GDF-1 gene.

### Example 5

### Preparation of antisera directed against GDF-1

Antibodies directed against GDF-1 can be used to characterize GDF-1 at the protein level. For this purpose, various portions of the GDF-1 protein have been overproduced in bacteria (Figure 9) using the T7-based expression vectors provided by Dr. F.W. Studier. Because the GDF-1 precursor is likely to be cleaved approximately 120 amino acids from the C-terminus, several of these overproduced proteins can be used as immunogens to obtain antibodies directed against the mature C-terminus fragment as well as against the presumed pro-region. Specifically, the GDF-1 fragments spanning amino acids 13 to 217 (which are fully contained within the pro- region) or amino acids 254-357 (which are fully contained within the mature C-terminal fragment) as well as the overproduced protein extending from amino acids 13 to 357, have been excised from preparative SDS polyacrylamide gels and can be used to immunize rabbits. Sera obtained from these rabbits following each boost can be tested by Western blot analysis [Burnette, Anal. Biochem. 112:195-203 (1981)] of extracts prepared from bacteria harboring the overproducing plasmids. This analysis can reveal whether antibodies have been produced that recognize the bacterially-produced immunogen. The animals can be boosted until a significant positive response is achieved as determined by this assay. To determine whether these antibodies also recognize nondenatured GDF-1, sense RNA derived from the full-length cDNA can be transcribed (from the T3 or T7 promoters of subclones in the Bluescript vector), capped, and translated *in vitro* in the presence of [³⁵S]methionine. The antisera can then be tested for the ability to immunoprecipitate these translation products.

### Example 6

### Purification of GDF-1 from mammalian cells

In order to obtain GDF-1 to assay for biological activity, the protein can be overproduced using the cloned cDNA. Because the pro- regions of the members of this superfamily appear to be necessary for the proper assembly of the active disulfide-linked dimers [Gray and Masqn, Science 247:1328-1330 (1990)], and because proper assembly and cleavage may not occur in bacteria, a mammalian cell line overproducing GDF-1 can be constructed. For this purpose, GDF-1 can be expressed in Chinese hamster ovary cells using the pMSXND expression vector [Lee and Nathans, J. Biol. Chem. 263:3521-3527 (1988)]. This vector contains a Mt-I promoter, a unique Xho I cloning site, splice and polyadenylation signals derived from SV40, a selectable marker for G418, and the murine dihydrofolate reductase (dhfr) gene under the control of the SV40 early promoter. The GDF-1 cDNA, truncated at the Hind III site in the 3' untranslated region, has been cloned downstream of the Mt-I promoter. The resulting construct, linearized at the unique Pvu I site (to enrich for integration events in this non-essential region), was transfected into CHO cells using the calcium phosphate method [Frost and Williams, Virology 91:39-50 (1978); van der Eb and Graham, Methods Enzymol. 65:826-839 (1980)]. G418-resistant clones can be grown in the presence of methotrexate to select for cells that amplify the dhfr gene and, in the process, co-amplify the adjacent GDF-1 gene. This vector and amplification scheme has been used in the past to construct a cell line in which one milligram of the desired protein was produced in seven 150 cm² tissue culture flasks [Lee and Nathans, J. Biol. Chem 263:3521-3527 (1988)]. In addition, because CHO cells can be maintained in a totally protein-free medium [Hamilton and Ham, in Vitro 13:537-547 (1977)], the desired secreted protein represented 10% of the total protein in the medium. This vector has also been made available to numerous other investigators, who have also overproduced their desired proteins in this manner [for example, see Colosi et al, Mol. Endocrinol. 2:579-586 (1988)].

Based on the results of *in vitro* translation experiments and on the known properties of other family members, it is likely that GDF-1 protein will be secreted into the medium. This can be verified by demonstrating the presence of GDF-1 in the conditioned medium of the overproducing cells by Western analysis. It also seems likely that the full length GDF-1 protein will be cleaved to generate the mature C-terminal fragment; indeed, such processing has been observed in the case of BMP-2a similarly overproduced in CHO cells [Wang et al, Proc. Natl. Acad. Sci., USA 87:2220-2224 (1990)]. Whether cleavage of GDF-1 takes place in the overproducing cells can be assessed by looking (by Western analysis) for the presence of a protein of the predicted size for the C-terminal fragment that reacts with antibodies directed against the C-terminal region but not with antibodies directed against the pro-region.

The mature GDF-1 protein can be purified from the conditioned medium of the producing cell line using standard protein separation techniques. An appropriate purification scheme can be empirically determined taking advantage of the known physical properties of other family members. For example, some of these proteins are known to have a high affinity for heparin [Ling et al, Proc. Natl. Acad. Sci. USA 82:7217-7221 (1985); Wang et al, Proc. Natl. Acad. Sci., USA 87:2220-2224 (1990)]. The final scheme can include an ion exchange chromatography step, a gel filtration step, and a reverse phase HPLC step. Each step of the purification can be monitored by electrophoresing column fractions on SDS polyacrylamide gels and identifying GDF-1 containing fractions by Western analysis. The purity at each step can be assessed by silver-staining of total proteins [Morrissy, Anal. Bioch. 117:307 (1981)]. The purified protein can be subjected to N-terminal amino acid sequencing to verify that the purified protein is GDF-1 and to precisely localize the site of cleavage from the precursor.

### Example 7

### Cloning and nucleotide sequence of the 3.0 kb GDF-1 transcript

To determine whether the 3.0 kb band represents an alternate transcript derived from the GDF-1 gene or a transcript derived from a different gene homologous to GDF-1, several cDNA libraries were constructed from poly A-selected adult mouse brain mRNA and screened with the 1.4 kb GDF-1 probe. From approximately one million recombinant phage screened from each of two separate oligo-dT primed cDNA libraries, a single clone (mBr-1) was isolated that hybridized with the GDF-1 probe at high stringency. Seven hybridizing clones (mBr-2 through mBr-8) were obtained by screening 0.6 million recombinant phage from a randon-primed cDNA library. An additional 0.7 million recombinant phage from a randon-primed cDNA library were screened with a probe derived from the 5' portion of clone mBr-7 to obtain clones mBr-9 through mBr-14. Based on partial nucleotide sequence analysis of the ends of the clones, these 14 could be aligned within a region spanning 2.7 kb (Figure 10a). The complete 2.7 kb cDNA sequence, obtained by determining the entire nucleotide sequence of clones mBr-1, mBr-2, and mBr-7, is shown in Figure lla. Sequence comparison showed that the previously-reported 1.4 kb sequence was essentially fully contained within the 2.7 kb sequence (from nucleotides 1311 to 2687) Within this region, the two sequences show three nucleotide differences. The sequence derived from clones mBr-2 and mBr-7 contains a C in place of T at position 1725, an A in place of T at position 1960, and a G in place of A at position 1974 compared to the sequence derived from a day 8.5 embryo cDNA clone. Although two of these differences represent third position changes that do not alter the predicted amino acid sequence, one of the differences changes the cysteine at codon 145 to a serine. For simplicity, the coding sequence corresponding to a cysteine at position 145 will be referred to as GDF-1a, and the sequence corresponding to a serine at position 145 will be referred to as GDF-1b. To determine whether the expression of GDF-1a and GDF-1b is specific for the respective tissues from which they were isolated, the nucleotide sequences of 5 independent clones isolated from a day 8.5 embryo cDNA library and 7 independent clones isolated from an adult brain cDNA library were determined in a limited region spanning the nucleotide positions at which GDF-1a and GDF-1b differ. The sequence analysis revealed that of the 5 embryonic clones, 3 corresponded to GDF-1a, and 2 corresponded to GDF-1b; of the 7 brain clones, 2 corresponded to GDF-1a, and 5 corresponded to GDF-1b. Hence, both GDF-1a and GDF-1b appear to be expressed both in day 8.5 embryos, where only the 1.4 kb mRNA species could be detected, and in the adult brain, where only the 3.0 kb mRNA species could be detected. GDF-1a and GDF-1b may represent allelic differences or two different genes.

Upstream of the GDF-1 coding region, the 2.7 kb sequence contained an additional 1310 bp not present in the 1.4 kb sequence, leaving a total of 1527 bp upstream of the initiating cpdon for GDF-1. Unexpectedly, within this upstream region was a second long open reading frame beginning with a putative initating methionine codon at nucleotide 74, extending for 350 codons, and terminating 404 nucleotides upstream of the GDF-1 initiating ATG. For simplicity, this second open reading frame will be hereafter referred to as mUOG-1 (upstream of GDF-1). Because of the presence of multiple stop codons in the region between mUOG-1 and mGDF-1, at least 4 frameshifts would be required to translate the two open reading frames as a single protein. A search of the NBRF and GenBank sequence databases with the predicted mUOG-1 amino acid sequence and with the entire upstream nucleotide sequence, respectively, revealed no significant homologies with known sequences. However, hydropathicity analysis of the predicted mUOG-1 amino acid sequence revealed multiple clusters (at least seven) of hydrophobic residues, reminiscent of membrane spanning domains (Figure 12). Particularly striking is the most distal of these clusters, which is immediately followed by a highly charged C-terminal region. Like certain other proteins with multiple membrane-spanning domains [for example, see Nathans et al, Cell 34:807 (1983); Dixon et al, Nature 321:75 (1986)], mUOG-1 does not contain an obvious N-terminal signal sequence.

### Example 8

### Isolation of the human GDF-1 gene

In order to carry out sequence comparisons to look for potentially signficant conserved regions in the GDF-1 mRNA and protein sequences, cDNA's encoding human GDF-1 were isolated using the murine GDF-1 probe. Three hybridizing clones (hBr-1 though hBr-3) were isolated from screening 0.6 million recombinant phage from a human adult cerebellum cDNA library (oligo dT-primed), and five clones (hBr-4 through hBr-8) were isolated from screening 1.4 million recombinant phage from a human fetal brain cDNA library (oligo dT/randon hexanucleotide-primed) (Figure 10b). Figure 11b shows the 2510 bp human cDNA sequence obtained by determining the entire nucleotide sequence of clone hBr-5 and the 5'-most 400 nucleotides of clones hBr-6, hBr-7, and hBr-8. The 3'-half of the sequence contains a long open reading frame beginning with an ATG codon at nucleotide 1347 and potentially encoding a protein of 372 amino acids with a molecular weight of 38,853. The predicted amino acid sequence shows significant similarity to murine GDF-1 (Figure 13a). Like the murine GDF-1 sequence, the human sequence contains a pair of basic residues (R-R) at amino acids 252-253, which presumably represents a site for proteolytic processing. Following the predicted cleavage site, the sequence contains all of the invariant and most of the highly conserved amino acids characteristic of all members of the TGF-β superfamily including the seven cysteine residues. The murine GDF-1 sequence and the human sequence are 87% identical in the region beginning with the first conserved cysteine and extending to the C-terminus and 69% identical thoughout the entire length of the protein. Because other members of the TGF-β superfamily show a much higher degree of sequence conservation across species [Cate et al, Cell 45:685 (1986); Mason et al, Nature 318:659 (1985); Forage et al, Proc. Natl. Acad. Sci., USA 83:3091 (1986); Derynck et al, Nature 316:701 (1985); Mason et al, Biochem. Biophys. Res. Commun. 135:957 (1986); Derynck et al, J. Biol. Chem. 261:4377 (1986); de Martin et al, EMBO J. 6:3673 (1987); ten Dijke et al, Proc. Natl. Acad. Sci., USA 85:4715 (1988); Derynck et al, EMBO J. 7:3737 (1988); Miller et al, Mol. Endocrinol. 3:1108 (1989); ibid, p. 1926; Dickinson et al, Genomics 6:505 (1990)], genomic Southern analysis was carried out to determine whether the murine and human sequences represent the same gene. As shown in Figure 14, both murine and human probes derived from the GDF-1 open reading frame hybridized to the same pattern of bands in human DNA, verifying that the human gene is indeed the homolog of murine GDF-1.

Like the murine sequence, the human sequence also contains a second long open reading frame potentially encoding 350 amino acids in the region upstream of the GDF-1 coding sequence. An alignment of this upstream open reading frame (hUOG-1) with that present in the murine sequence showed that the upstream open reading frame is even more highly conserved than that for GDF-1 (Figure 13b), with the overall amino acid sequence identity between mUOG-1 and hUOG-1 being 81%. Although the open reading frames for both mUOG-1 and hUOG-1 extend upstream of the putative initiating methionine to the very 5' ends of the sequences, two lines of reasoning suggest that these may be the true initiation codons. First, multiple cDNA's primed by random hexanucleotides at various distances from the 3' end terminated very close to the 5' ends of both the murine and human sequences (Figure 10). Second, the murine and human nucleotide and amino acid sequences show much less conservation upstream of the putative initiation codon for UOG-1 than in the coding sequence itself (Figure 13c). In contrast to the high degree of conservation observed between mUOG-1 and hUOG-1 and hUOG-1 and between mGDF-1 and hGDF-1, the intervening spacer region and the putative 5' and 3' untranslated regions show much less similarity between the murine and human sequences. This selective conservation of the two open reading frames is most clearly evident in a DIAGON plot comparing the murine and human nucleotide sequences (Figure 13c). The two sequences begin to diverge in the intervening spacer region precisely after the stop codons for UOG-1 and in the 3' untranslated region 2 nucleotides following the stop codons for GDF-1. Moreover, the intervening spacer region in the murine sequence is 401 nucleotides in length whereas the corresponding region in the human sequence is only 269 nucleotides in length. The conservation of the amino acid sequence of UOG-1 is also evident in the non-random pattern of nucleotide differences between the murine and human sequences spanning the UOG-1 open reading frames. Of the 209 nucleotide differences in this region, 57 represent first position differences, 29 represent second position differences, and 123 represent third position differences; of the 123 third position differences, 89 do not result in differences in the predicted amino acid sequence.

All publication mentioned hereinabove are hereby incorporated by reference.

While the foregoing invention has been described in some detail for purposes of clarity and understanding, it will be appreciated by one skilled in the art from a reading of this disclosure that various changes in form and detail can be made without departing from the true scope of the invention.

## Claims (Claims for the following Contracting State(s): AT, BE, CH, LI, DE, DK, FR, GB, GR, IT, LU, NL, SE)

1. A DNA segment encoding a mammalian GDF-1 protein being a mouse protein or a human protein of the sequence shown in Figures 2 and 11A for the mouse GDF-1 or in Figure 11B for human GDF-1 or encoding a mammalian UOG-1 protein being a mouse protein or human protein of the sequence shown in Figure 11A for the mouse UOG-1 or Figure 11B for the human UOG-1 or encoding a peptide including an epitope of any of the foregoing proteins, or a DNA fragment of at least 15 nucleotides of a said DNA segment or a complementary DNA segment.

2. A mammalian GDF-1 or UOG-1 protein (as defined in Claim 1), substantially free of proteins with which it is naturally non-covalently associated, or a peptide including an epitope of a said protein.

3. The protein according to Claim 2, wherein said protein is chemically synthesised.

4. The protein according to Claim 2 or Claim 3, wherein said protein is unglycosylated.

5. A recombinant DNA molecule comprising:
i) a DNA segment as claimed in Claim 1; and
ii) a vector.

6. A procaryotic or eucaryotic host cell stably transformed with said recombinant DNA molecule according to Claim 5.

7. A method of producing a recombinant GDF-1 or UOG-1 protein (as defined in Claim 1), comprising culturing said host cell according to Claim 6 under conditions such that said segment is expressed and said GDF-1 or UOG-1 protein thereby produced, and isolating said GDF-1 or UOG-1 protein.

8. A polyclonal or monoclonal antibody immunologically reactive with a protein or peptide as claimed in any one of Claims 2 to 4.

## Claims (Claims for the following Contracting State(s): ES)

1. A process for the production of a DNA segment or a DNA fragment comprising producing a DNA segment encoding a mammalian GDF-1 protein being a mouse protein or a human protein of the sequence shown in Figures 2 and 11A for the mouse GDF-1 or in Figure 11B for human GDF-1 or encoding a mammalian UOG-1 protein being a mouse protein or human protein of the sequence shown in Figure 11A for the mouse UOG-1 or Figure 11B for the human UOG-1 or encoding a peptide including an epitope of any of the foregoing proteins, or a DNA fragment of at least 15 nucleotides of a said DNA segment or a complementary DNA segment.

2. A process for the production of a mammalian GDF-1 or UOG-1 protein, as defined in Claim 1, or a peptide thereof, comprising producing a said protein substantially free of proteins with which it is naturally non-covalently associated, or a peptide including an epitope of a said protein.

3. A method according to Claim 2, wherein said protein is chemically synthesised.

4. A method according to Claim 2 or Claim 3, wherein said protein is unglycosylated.

5. A method for producing a recombinant DNA molecule characterised in that said molecule comprises:
i) a DNA segment as defined in Claim 1; and
ii) a vector.

6. A method of transforming a procaryotic or eucaryotic host cell comprising stably transforming said cell with said recombinant DNA molecule as defined in Claim 5.

7. A method of producing a recombinant GDF-1 or UOG-1 protein (as defined in Claim 1), comprising culturing said host cell according to Claim 6 under conditions such that said segment is expressed and said GDF-1 or UOG-1 protein thereby produced, and isolating said GDF-1 or UOG-1 protein.

8. A method for producing polyclonal or monoclonal antibody immunologically reactive with a protein or peptide as claimed in any one of Claims 1 to 4, comprising immunising animal with a said protein or peptide and isolating a said antibody.

9. A DNA segment encoding a mammalian GDF-1 protein being a mouse protein or a human protein of the sequence shown in Figures 2 and 11A for the mouse GDF-1 or in Figure 11B for human GDF-1 or encoding a mammalian UOG-1 protein being a mouse protein or human protein of the sequence shown in Figure 11A or Figure 11B for UOG-1 or encoding a peptide including an epitope of any of the foregoing proteins, or a DNA fragment of at least 15 nucleotides of a said DNA sequence or a complementary DNA to a said DNA fragment.

10. A mammalian GDF-1 or UOG-1 protein (as defined in Claim 9), substantially free of proteins with which it is naturally non-covalently associated, or a peptide including an epitope of a said protein.

11. The protein according to Claim 10, wherein said protein is chemically synthesised.

12. The protein according to Claim 10 or Claim 11, wherein said protein is unglycosylated.

13. A recombinant DNA molecule comprising:
i) a DNA segment as claimed in Claim 9; and
ii) a vector.

14. A procaryotic or eucaryotic host cell stably transforming with said recombinant DNA molecule according to Claim 13.

15. A polyclonal or monoclonal antibody immunologically reactive with a protein or peptide as claimed in any one of Claims 10 to 12.

## Patentansprüche (Patentansprüche für folgende(n) Vertragsstaat(en): AT, BE, CH, LI, DE, DK, FR, GB, GR, IT, LU, NL, SE)

1. DNA-Segment, das ein Säuger-GDF-1-Protein kodiert, das ein Maus-Protein oder ein humanes Protein mit der in den Figuren 2 und 11A für das Maus-GDF-1 oder in Figur 11B für humanes GDF-1 gezeigten Sequenz ist, oder welches ein Säuger-UOG-1-Protein kodiert, das ein Maus-Protein oder ein humanes Protein mit der in Figur 11A für das Maus-UOG-1 oder in Figur 11B für das humane UOG-1 gezeigten Sequenz ist, oder welches ein Peptid kodiert, das ein Epitop irgendeines der vorhergehenden Proteine einschließt, oder ein DNA-Fragment mit mindestens 15 Nukleotiden des DNA-Segmentes oder ein komplementäres DNA-Segment.

2. Säuger-GDF-1- oder UOG-1-Protein (wie in Anspruch 1 definiert), das im wesentlichen frei von Proteinen ist, mit denen es natürlicherweise nicht-kovalent assoziiert ist, oder ein Peptid, das ein Epitop des Proteins einschließt.

3. Protein nach Anspruch 2, wobei das Protein chemisch synthetisiert ist.

4. Protein nach Anspruch 2 oder 3, wobei das Protein unglykosyliert ist.

5. Rekombinantes DNA-Molekül umfassend:
i) ein DNA-Segment wie es in Anspruch 1 beansprucht ist; und
ii) einen Vektor.

6. Prokaryontische oder eukaryontische Wirtszelle, die mit dem rekombinanten DNA-Molekül nach Anspruch 5 stabil transformiert ist.

7. Verfahren zur Herstellung eines rekombinanten GDF-1- oder UOG-1-Proteins (wie in Anspruch 1 definiert), bei dem man die Wirtszelle nach Anspruch 6 unter Bedingungen kultiviert, die zur Expression des Segmentes und damit zur Produktion des GDF-1- oder UOG-1-Proteins führen, und man das GDF-1- oder UOG-1-Protein isoliert.

8. Polyklonaler oder monoklonaler Antikörper, der mit einem Protein oder einem Peptid immunologisch reagiert, wie es in einem der Ansprüche 2 bis 4 beansprucht ist.

## Patentansprüche (Patentansprüche für folgende(n) Vertragsstaat(en): ES)

1. Verfahren zur Herstellung eines DNA-Segments oder eines DNA-Fragments, bei dem man ein DNA-Segment herstellt, das ein Säuger-GDF-1-Protein kodiert, welches ein Maus-Protein oder ein humanes Protein mit der in den Figuren 2 und 11A für das Maus-GDF-1 oder in Figur 11B für humanes GDF-1 gezeigten Sequenz ist, oder welches ein Säuger-UOG-1-Protein kodiert, das ein Maus-Protein oder ein humanes Protein mit der in Figur 11A für das Maus-UOG-1 oder in Figur 11B für das humane UOG-1 gezeigten Sequenz ist, oder welches ein Peptid kodiert, das ein Epitop irgendeines der vorhergehenden Proteine einschließt, oder ein DNA-Fragment mit mindestens 15 Nukleotiden des DNA-Segmentes oder ein komplementäres DNA-Segment.

2. Verfahren zur Herstellung eines Säuger-GDF-1- oder UOG-1-Proteins, wie es in Anspruch 1 definiert ist oder eines Peptids davon, bei dem man das Protein im wesentlichen frei von Proteinen herstellt, mit denen es natürlicherweise nicht-kovalent assoziiert ist, oder eines Peptids, das ein Epitop dieses Proteins einschließt.

3. Verfahren nach Anspruch 2, bei dem man das Protein chemisch synthetisiert.

4. Verfahren nach Anspruch 2 oder 3, bei dem das Protein unglykosyliert ist.

5. Verfahren zur Herstellung eines rekombinanten DNA-Moleküls, dadurch gekennzeichnet, daß das Molekül
i) ein wie in Anspruch 1 definiertes DNA-Segment; und
ii) einen Vektor
umfaßt.

6. Verfahren zur Transformation einer prokaryontischen oder eukaryontischen Wirtszelle, bei dem man die Zelle mit dem rekombinanten DNA-Molekül, wie es in Anspruch 5 definiert ist, stabil transformiert.

7. Verfahren zur Herstellung eines rekombinanten GDF-1- oder UOG-1-Proteins (wie in Anspruch 1 definiert), bei dem man die Wirtszelle nach Anspruch 6 unter Bedingungen kultiviert, die zu einer Expression des Segmentes und damit zur Produktion des GDF-1- oder UOG-1-Proteins führen, und man das GDF-1- oder UOG-1-Protein isoliert.

8. Verfahren zur Herstellung eines polyklonalen oder monoklonalen Antikörpers, der mit einem Protein oder Peptid, wie es in einem der Ansprüche 1 bis 4 beansprucht ist, immunologisch reagiert, bei dem man ein Tier mit einem solchen Protein oder Peptid immunisiert und den Antikörper isoliert.

9. DNA-Segment, das ein Säuger-GDF-1-Protein kodiert, das ein Maus-Protein oder ein humanes Protein mit der in den Figuren 2 und 11A für das Maus-GDF-1 oder in Figur 11B für humanes GDF-1 gezeigten Sequenz ist, oder welches ein Säuger-UOG-1-Protein kodiert, das ein Maus-Protein oder ein humanes Protein mit der in Figur 11A oder Figur 11B für UOG-1 gezeigten Sequenz ist, oder welches ein Peptid kodiert, das ein Epitop irgendeines der vorhergehenden Proteine einschließt, oder ein DNA-Fragment mit mindestens 15 Nukleotiden der DNA-Sequenz oder eine zu dem DNA-Fragment komplementäre DNA.

10. Säuger-GDF-1- oder UOG-1-Protein (wie in Anspruch 9 definiert), das im wesentlichen frei von Proteinen ist, mit denen es natürlicherweise nicht-kovalent assoziiert ist, oder ein Peptid, das ein Epitop des Proteins einschließt.

11. Protein nach Anspruch 10, wobei das Protein chemisch synthetisiert ist.

12. Protein nach Anspruch 10 oder 11, wobei das Protein unglykosyliert ist.

13. Rekombinantes DNA-Molekül, umfassend:
i) ein DNA-Segment wie es in Anspruch 9 beansprucht ist; und
ii) einen Vektor.

14. Prokaryontische oder eukaryontische Wirtszelle, die mit dem rekombinanten DNA-Molekül nach Anspruch 13 stabil transformiert ist.

15. Polyklonaler oder monoklonaler Antikörper, der mit einem Protein oder Peptid immunologisch reagiert, wie es in einem der Ansprüche 10 bis 12 beansprucht ist.

## Revendications (Revendications pour l'(les) Etat(s) contractant(s) suivant(s): AT, BE, CH, LI, DE, DK, FR, GB, GR, IT, LU, NL, SE)

1. Segment d'ADN codant pour une protéine GDF-1 de mammifère, qui est une protéine de souris ou une protéine humaine ayant la séquence présentée sur les Figures 2 et 11A pour la GDF-1 de souris ou sur la Figure 11B pour la GDF-1 humaine, ou codant pour une protéine UOG-1 de mammifère, qui est une protéine de souris ou une protéine humaine ayant la séquence présentée sur la Figure 11A pour la UOG-1 de souris ou sur la Figure 11B pour la UOG-1 humaine, ou codant pour un peptide comprenant un épitope de l'une des protéines précédentes, ou un fragment d'ADN d'au moins 15 nucléotides d'un tel segment d'ADN, ou un segment d'ADN complémentaire.

2. Protéine GDF-1 ou UOG-1 de mammifère (telle que définie dans la revendication 1), pratiquement exempte des protéines avec lesquelles elle est associée naturellement de façon non covalente, ou peptide comprenant un épitope d'une telle protéine.

3. Protéine selon la revendication 2, dans laquelle ladite protéine est synthétisée chimiquement.

4. Protéine selon la revendication 2 ou la revendication 3, dans laquelle ladite protéine est non glycosylée.

5. Molécule d'ADN recombiné comprenant :
i) un segment d'ADN selon la revendication 1 ; et
ii) un vecteur.

6. Cellule hôte procaryote ou eucaryote transformée de façon stable avec ladite molécule d'ADN recombiné selon la revendication 5.

7. Procédé de production d'une protéine GDF-1 ou UOG-1 recombinée (telle que définie dans la revendication 1), comprenant la culture de ladite cellule hôte selon la revendication 6 dans des conditions telles que ledit segment soit exprimé et que lesdites protéines GDF-1 ou UOG-1 soient ainsi produites, et l'isolation desdites protéines GDF-1 ou UOG-1.

8. Anticorps polyclonal ou monoclonal réactif, du point de vue immunologique, avec une protéine ou un peptide selon l'une des revendications 2 à 4.

## Revendications (Revendications pour l'(les) Etat(s) contractant(s) suivant(s): ES)

1. Procédé de production d'un segment d'ADN ou d'un fragment d'ADN comprenant la production d'un segment d'ADN codant pour une protéine GDF-1 de mammifère qui est une protéine de souris ou une protéine humaine ayant la séquence représentée sur les figures 2 et 11A pour la GDF-1 de souris ou sur la figure 11B pour la GDF-1 humaine ou codant pour une protéine UOG-1 de mammifère qui est une protéine de souris ou une protéine humaine ayant la séquence représentée sur la figure 11A pour l'UOG-1 de souris ou la figure 11B pour l'UOG-1 humaine ou codant pour un peptide comprenant un épitope de l'une des protéines précédentes ou un fragment d'ADN contenant au moins 15 nucléotides dudit segment d'ADN ou un segment d'ADN complémentaire.

2. Procédé pour la production de GDF-1 ou UOG-1 de mammifère, tels que définis dans la revendication 1, ou un de leurs peptides comprenant la production de ladite protéine pratiquement exempte des protéines qui leur sont naturellement associées de manière non-covalente ou un peptide comprenant un épitope de ladite protéine.

3. Procédé selon la revendication 2, selon lequel la protéine est synthétisée chimiquement.

4. Procédé selon l'une des revendications 2 ou 3, selon lequel la protéine est non-glycosylée.

5. Procédé de production d'une molécule d'ADN recombiné, caractérié par le fait que la molécule comprend :
i) un segment d'ADN tel que défini dans la revendication 1 ; et
ii) un vecteur.

6. Procédé de transformation d'une cellule hôte procaryote ou eucaryote comprenant la transformation stable de ladite cellule avec une molécule d'ADN recombiné telle que définie dans la revendication 5.

7. Procédé de production de protéine GDF-1 ou UOG-1 recombiné (telle que définie dans la revendication 1), comprenant la culture de ladite cellule hôte selon la revendication 6 dans des conditions telles que ledit segment soit exprimé et que lesdites protéines GDF-1 ou UOG-1 soient ainsi produites et l'isolation desdites protéines GDF-1 ou UOG-1.

8. Procédé de préparation d'un anticorps polyclonal ou monoclonal réactif du point de vue immunologique avec une protéine ou un peptide tel que revendiqué dans l'une des revendications 1 à 4, comprenant le fait d'immuniser un animal avec ladite protéine ou ledit peptide et d'isoler ledit anticorps.

9. Segment d'ADN codant pour une protéine GDF-1 de mammifère, qui est une protéine de souris ou une protéine humaine ayant la séquence présentée sur les figures 2 et 11A pour la GDF-1 de souris ou sur la figure 11B pour la GDF-1 humaine, ou codant pour une protéine UOG-1 de mammifère, qui est une protéine de souris ou une protéine humaine ayant la séquence présentée sur la figure 11A pour la UOG-1 de souris ou sur la figure 11B pour la UOG-1 humaine, ou codant pour un peptide comprenant un épitope de l'une des protéines précédentes, ou un fragment d'ADN d'au moins 15 nucléotides d'un tel segment d'ADN, ou un segment d'ADN complémentaire de ce fragment d'ADN.

10. Protéine GDF-1 ou UOG-1 de mammifère (telle que définie dans la revendication 9), pratiquement exempte des protéines avec lesquelles elle est associée naturellement de façon non covalente, ou peptide comprenant un épitope d'une telle protéine.

11. Protéine selon la revendication 10, dans laquelle ladite protéine est synthétisée chimiquement.

12. Protéine selon la revendication 10 ou la revendication 11, dans laquelle ladite protéine est non glycosylée.

13. Molécule d'ADN recombiné comprenant :
i) un segment d'ADN selon la revendication 9 ; et
ii) un vecteur.

14. Cellule hôte procaryote ou eucaryote transformée de façon stable avec ladite molécule d'ADN recombiné selon la revendication 13.

15. Anticorps polyclonal ou monoclonal réactif, du point de vue immunologique, avec une protéine ou un peptide selon l'une des revendications 10 à 12.
